# EUROPÄISCHE PATENTSCHRIFT

(11) **EP 3 958 765 B1**
(45) Veröffentlichungstag und Bekanntmachung des Hinweises auf die Patenterteilung: **04.06.2025**
(21) Anmeldenummer: 20732491.4
(22) Anmeldetag: 08.04.2020
(51) Int. Cl.: A61B 17/70, A61B 17/88, A61B 17/00, A61B 90/00, A61B 50/20, A61B 50/30, A61B 50/33, A61B 50/00, B25B 23/00

(54) **SET ZUR DORSALEN WIRBELFUSION SOWIE HANDGRIFF FÜR EIN MEDIZINISCHES WERKZEUG**
SET FOR DORSAL SPINAL FUSION AND HANDLE FOR A MEDICAL TOOL
ENSEMBLE POUR LA FUSION DES VERTÈBRES DORSALES ET POIGNÉE POUR UN OUTIL MÉDICAL

(30) Priorität: 23.04.2019 DE 102019110442
(43) Veröffentlichungstag der Anmeldung: 02.03.2022
(73) Patentinhaber: Richard Wolf GmbH, 75438 Knittlingen (DE)
(72) Erfinder: LUDWIG, Volker, 65344 Eltville (DE); RÖBLING, Christian, 65344 Eltville (DE)
(74) Vertreter: Friderichs, Gunther
(86) Internationale Anmeldenummer: PCT/EP2020/059960
(87) Internationale Veröffentlichungsnummer: WO 2020/216616

(56) Entgegenhaltungen:
- WO-A1-2013/178932
- US-A1- 2006 241 627
- US-A1- 2015 174 754
- US-A1- 2016 346 017
- US-A1- 2017 189 082
- US-A1- 2017 224 399
- US-A1- 2019 022 833

## Beschreibung

### Gebiet der Erfindung

Die Erfindung betrifft ein Set zur dorsalen Wirbelfusion, sowie einen dafür ausgebildeten Handgriff, welcher zur Aufnahme eines Antriebs eines Schraubendrehers ausgebildet ist.

### Hintergrund der Erfindung

Für die dorsale Wirbelfusion werden in der Praxis insbesondere Schrauben verwendet, deren Kopf eine Tulpe mit einer Durchführung für eine Fusionsstange aufweist.

Die Schrauben werden als Knochenschrauben in den Wirbelkörper eingebracht und sodann wird eine Stange durch die Öffnung in den Tulpen der Schraubenköpfe geschoben. Nach Befestigen der Stange mittels eines Verschlusses sind die Wirbelkörper über die Stangen verblockt.

Hierfür wird in der Praxis zumeist ein umfangreiches Instrumentarium verwendet. In der Regel wird zunächst mit einer Zugangsnadel durch die Haut punktiert und so der Kanal markiert.

Sodann wird ein Führungsdraht eingesetzt, mittels eines Dilators das Weichgewebe aufgeweitet und die Schrauben eingebracht, welche sodann mit den Stäben verbunden werden.

Zum Abschluss können die Stäbe mit jeweils einem Verschluss fixiert werden. Es kann sich dabei insbesondere um einen Verschluss mit einer Mutter mit einem abbrechbaren Kopfstück handeln, welches beim Erreichen eines bauartbedingt vorgegebenen Drehmoments abbricht. Als Antrieb, insbesondere für die verwendeten Schraubendreher, können abnehmbare Griffe verwendet werden. Insbesondere hat sich in der Praxis ein Vierkantantrieb an den Schraubendrehern etabliert, welcher werkzeuglos an einem Handgriff verrastet werden kann.

Da das Einbringen der Schrauben mühsam ist, bevorzugen manche Anwender Ratschengriffe.

Ein derartiger Ratschengriff ist beispielsweise aus dem Dokument WO 2013/178932 A1 bekannt.

Die aus der Praxis bekannten Sets, welche zumindest einen Teil der für die dorsalen Wirbelfusion benötigten Instrumente umfassen, sind zumeist vollständig als Mehrweginstrumentarium ausgebildet. Dies hat zwangsläufig eine kostspielige und aufwändige Sterilisation des Instrumentariums nach jeder Verwendung zur Folge.

Bei aus der Praxis bekannten Instrumentarien, insbesondere mit vorstehend genanntem Ratschengriff, ist allerdings zumeist die Aufnahme für den Antrieb des Werkzeugs derartig aufwändig ausgebildet, dass die Griffe recht kostspielig herzustellen sind.

Eine Verwendung eines derartigen Griffes, bei dem insbesondere die Hülse zur Aufnahme des Antriebs aus Metall ausgebildet ist, als Teil eines Einweginstrumentariums macht daher keinen Sinn.

Zur medizinischen Verwendung bestimmte Werkzeuge mit einem Handgriff sind aus den Dokumenten US 2017/224399 A1, US 2015/174754 A1, US 2006/241627 A1, US 2017/189082 A1 und US 2016/346017 A1 bekannt.

Ratschengriffe sind aus den Dokumenten US 2019/022833 A1 und WO 2013/178932 A1 bekannt.

### Aufgabe der Erfindung

Der Erfindung obliegt demgegenüber die Aufgabe, ein Set zur dorsalen Wirbelfusion sowie einen dafür verwendbaren Handgriff bereit zu stellen, welches bzw. welcher einfach und kostengünstig herzustellen ist und insbesondere die Bereitstellung eines Einweginstrumentariums ermöglicht.

### Zusammenfassung der Erfindung

Die Aufgabe der Erfindung wird bereits durch ein Set zur dorsalen Wirbelfusion sowie durch einen Handgriff für ein medizinisches Werkzeug nach Anspruch 1 gelöst.

Bevorzugte Ausführungsformen und Weiterbildungen der Erfindung sind Gegenstand der abhängigen Ansprüche.

Die Erfindung betrifft ein Set zur dorsalen Wirbelfusion, welches insbesondere als Einweginstrumentarium ausgebildet ist. Die in dem Set vorhandenen Werkzeuge können insbesondere in einer sterilen Verpackung, beispielsweise in einer aufreißbaren Blisterverpackung, angeordnet sein.

Das Set umfasst zumindest einen abnehmbaren Handgriff aus Kunststoff sowie zumindest einen mit dem abnehmbaren Handgriff werkzeuglos koppelbaren Schraubendreher, wobei eine Aufnahme des Handgriffes für den Schraubendreher aus Kunststoff besteht.

Im Unterschied zu bekannten Handgriffen mit einer Aufnahme, die in der Regel eine mit Kunststoff umspritzte Metallhülse umfasst, ist es durch die Erfindung möglich, die Aufnahme für den Werkzeugantrieb einstückig mit dem Griff auszubilden.

Es hat sich herausgestellt, dass so kostengünstig ein geeigneter Griff herstellbar ist. Insbesondere bei Wahl eines geeigneten Kunststoffes reicht eine aus Kunststoff ausgebildete Aufnahme für die Verwendung des Griffes im Rahmen eines Einweginstrumentariums aus. Es besteht also insbesondere die Aufnahme für den Antrieb des Schraubendrehers aus Kunststoff. Diese kann z.B. polygonförmig, insbesondere vierkantförmig, ausgebildet sein. Der Handgriff besteht vorzugsweise aus einem Polymer, insbesondere aus einem Thermoplast. Dies ermöglicht eine Herstellung im Spritzgussverfahren.

Als besonders geeignet hat sich insbesondere eine Polyacrylamid erwiesen.

Insbesondere kann für den Handgriff ein faserverstärkter Kunststoff, insbesondere ein glasfaserverstärktes Polyacrylamid, verwendet werden.

Gemäß der Erfindung umfasst der Handgriff eine Werkzeugaufnahme aus Kunststoff, wobei der Antrieb eines Werkzeugs mittels eines federnden Hakens aus Kunststoff, welcher integraler Bestandteil des Handgriffes ist, verrastbar ist.

Zum Einrasten des Werkzeugs wird ein federnder L-förmig ausgebildeter Haken verwendet, welcher zusammen mit dem Hauptgehäuse des Handgriffes oder einem Bestandteil des Handgriffes einstückig ausgebildet ist.

Der Haken federt allein durch die elastischen Eigenschaften des Kunststoffmaterials. Auf eine separate Feder kann so verzichtet werden.

Der federnde Haken ist integraler Bestandteil eines Hauptgehäuses des Griffes oder integraler Bestandteil der Hülse eines Ratschenmechanismus.

Der Handgriff ist vorzugsweise als länglicher Griff ausgebildet.

Gemäß einer Ausführungsform der Erfindung ist sowohl an einer Langseite als auch an einer Schmalseite eine Werkzeugaufnahme vorhanden.

Der Griff kann so, je nach Einsatzzweck und Präferenz, verwendet werden, um mit dem Werkzeug eine T-förmige Anordnung zu bilden oder, angekoppelt an seiner Schmalseite, entsprechend seiner Haupterstreckungsrichtung axial zum Werkzeug ausgerichtet sein.

Die T-förmige Anordnung ermöglicht das Aufbringen eines möglichst hohen Drehmoments, wohingegen die axiale Ausrichtung bei vielen Anwendungen ein schnelleres Ein- oder Herausdrehen sowie eine exaktere Winkelführung ermöglicht.

Gemäß der Erfindung ist der Handgriff als Ratschengriff ausgebildet.

Die Erfindung sieht insbesondere vor, dass ein derartiger Ratschengriff ein Kunststoffzahnrad umfasst, welches in das Hauptgehäuse eingesetzt ist und in welches eine Sperrklinke greift, die die Drehung des Kunststoffzahnrads in eine Drehrichtung blockiert.

So kann ein einfach ausgestalteter Ratschenmechanismus bereitgestellt werden, welcher aus nur wenigen Teilen besteht.

Erfindungsgemäß ist der Ratschenmechanismus nicht umschaltbar ausgebildet. Zum Ändern der Drehrichtung kann das Werkzeug von zwei verschiedenen Seiten in die Aufnahme eingerastet werden.

Das Zahnrad ist insbesondere integraler Bestandteil der Werkzeugaufnahme.

Insbesondere ist das Zahnrad Teil einer Hülse, welche zumindest einen, vorzugsweise zwei federnde Haken umfasst, mit dem bzw. denen das Werkzeug, insbesondere der Schraubendreher, verrastet werden kann.

Das Set kann des Weiteren zumindest eine auf eine Knochenschraube aufsetzbare Führungshülse, einen Dilator, einen Gegenhalter für eine Führungshülse, einen Halter für einen Fusionsstab und/oder einen Flankenbrecher für die Tulpe über eine Fusionsschraube umfassen.

Der Halter für den Fusionsstab ist vorzugsweise mit dem Handgriff koppelbar. So kann ein besonders kompakt ausgebildeter Halter bereitgestellt werden.

Gemäß einer Ausführungsform der Erfindung umfasst der Flankenbrecher für die Tulpe der Fusionsschraube den Gegenhalter für die Führungshülse.

Insbesondere kann der Flankenbrecher ein Ende mit einem Antrieb, beispielsweise einem Sechskant und ein anderes Ende mit einem Kanal umfassen, in welchen die Flanke eingeführt und sodann abgebrochen werden kann.

So wird ein Werkzeug mit einer Doppelfunktionalität bereitgestellt, was die Anzahl der benötigten Werkzeuge reduziert und eine preiswertere Bereitstellung des Sets ermöglicht.

Die Erfindung betrifft des Weiteren auch vorstehend beschriebenen Flankenbrecher, unabhängig davon, ob er Teil des vorstehend beschriebenen Sets ist.

Weiter betrifft die Erfindung einen Handgriff für ein medizinisches Werkzeug, welcher für vorstehend beschriebenes Set ausgebildet ist.

Gemäß der Erfindung besteht der Handgriff, also zumindest das Hauptgehäuse des Handgriffes, aus Kunststoff und der Handgriff umfasst einen Ratschenmechanismus mit einem Kunststoffzahnrad, in welches eine Sperrklinke greift, die eine Drehung in eine Drehrichtung blockiert.

Das Zahnrad ist seinerseits Teil einer Werkzeugaufnahme, in welche der Antrieb eines Werkzeugs, insbesondere eines Schraubendrehers, verrastbar ist.

Das Zahnrad ist insbesondere Teil einer Hülse, welche die Werkzeugaufnahme umfasst.

Vorzugsweise umfasst die Hülse, wie vorstehend beschrieben, auch zumindest einen federnden Haken zum Verrasten des Werkzeugs.

Der federnde Haken ist insbesondere integraler Bestandteil der Hülse. Insbesondere kann der Haken zusammen mit der Hülse als einstückiges Spritzgussbauteil bereitgestellt sein.

Bei einer Weiterbildung der Erfindung umfasst das Set zur dorsalen Wirbelfusion auch eine Nadel, insbesondere eine Zugangsnadel.

Eine Zugangsnadel umfasst eine Hohlnadel, welche von einer Innennadel trennbar ist.

Ein derartiges medizinisches Instrument wird auch das Trokar bezeichnet. Der Trokar ist ein Instrument, mit dessen Hilfe in der minimalinvasiven Chirurgie scharf oder stumpf ein Zugang zu einer Körperhöhle geschaffen und durch die Hohlnadel, den sog. Tubus offengehalten wird. Die Innennadel ist ein Stift, der in dem Tubus sitzt, und deren Spitze die Öffnung des Tubus verschließt.

Die Innennadel wird nach dem Einbringen des Trokar herausgezogen, ist also vom Trokar trennbar.

Die Nadel, insbesondere die Hohlnadel, umfasst eine Nut zum Eingriff des federnden Hakens.

Insbesondere umfasst das Set einen weiteren Griff für die Nadel.

Dieser Griff kann eine andere ergonomische Ausgestaltung als vorstehend beschriebener Griff haben.

Der Griff dient der Positionierung der Zugangsnadel.

Bei einer Weiterbildung der Erfindung umfasst der Griff eine Kupplung für eine Verlängerung, insbesondere eine abgewinkelte Verlängerung.

Durch die Verlängerung kann die Zugangsnadel unter Röntgenkontrolle positioniert werden, ohne dass der Benutzer in den Strahlengang greift.

Als Verlängerung kann insbesondere vorstehend beschriebener Gegenhalter/Flankenbrecher verwendet werden.

Die Kupplung kann insbesondere als Sechskant auf der proximalen Seite des Griffes ausgebildet sein.

Die Verrastung wird durch einen Haken bewirkt, welcher integraler Bestandteil des Handgriffes, insbesondere eines Hauptgehäuses des Handgriffes, ist.

Wie vorstehend beschrieben, kann insbesondere das Hauptgehäuse des Handgriffes oder ein Bauteil des Handgriffes, wie beispielsweise die vorstehend beschriebene Hülse des Ratschenmechanismuses als einstückiges Spritzgussbauteil zusammen mit dem federnden Haken ausgebildet sein.

Der Handgriff ist insbesondere derart ausgebildet, dass dieser in zwei um 90° verdrehten Positionen mit dem Werkezeug verbunden sein kann.

In axialer Ausrichtung zu dem Werkzeug kann der Handgriff auch als bloße Verlängerung eines Werkezeugs, insbesondere des Sets zur dorsalen Wirbelfusion, und nicht als Antrieb zum Drehen verwendet werden.

Gemäß einer Ausführungsform der Erfindung umfasst die Aufnahme an der Langseite einen Ratschenmechanismus.

Gemäß einer weiteren Ausführungsform der Erfindung ist der Handgriff zur Kopplung mit einer Nadel, insbesondere einer Zugangsnadel, ausgebildet.

Hierzu kann der Griff eine Durchführung für die Nadel umfassen.

Eine mit dem Griff gekoppelte Nadel umfasst eine Nut, in welcher der federnde Haken des Griffes verrastet.

Insbesondere umfasst eine Hohlnadel die Nut. Die Hohlnadel kann so auf den Griff aufgerastet und vom Griff abgetrennt werden.

Eine Innennadel kann vorzugsweise proximal aus dem Griff und der mit dem Griff gekoppelten Hohlnadel gezogen werden.

Die Innennadel kann einen Halter umfassen, der seinerseits mit dem Griff koppelbar ist, insbesondere über ein Gewinde in der Durchführung für die Nadel.

Bei einer Weiterbildung der Erfindung umfasst der Handgriff auf einer proximalen Seite eine Kupplung für eine Verlängerung, insbesondere eine abgewinkelte Verlängerung wie beispielsweise vorstehend beschriebenen Gegenhalter.

Der Griff für die Nadel ist von seiner Form her vorzugsweise derart ausgebildet, um, bezogen auf die Nadel, von der Seite gegriffen zu werden. Hierzu kann der Griff eine zylindrische oder bauchige Formhaben.

### Kurzbeschreibung der Zeichnungen

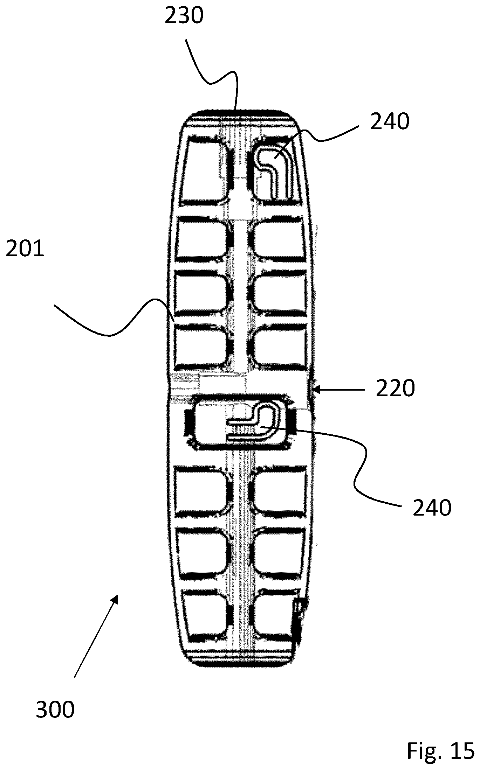

Die Erfindung soll im Folgenden Bezug nehmend auf ein in den Zeichnungen Fig. 1 bis Fig. 18 dargestelltes Ausführungsbeispiel näher erläutert werden.
Fig. 1 zeigt in der Draufsicht ein Set zur dorsalen Wirbelfusion.
Fig. 2 zeigt das Set in einer Seitenansicht auf die Langseite und Fig. 3 zeigt das Set in einer Seitenansicht auf die Schmalseite.
Fig. 4 zeigt die in dem Set beispielhaft vorhandenen Instrumente.
Fig. 5 ist eine Darstellung des in dem Set enthaltenen Flankenbrechers.
Fig. 6 und Fig. 7 sind perspektivische Darstellungen einer Schraube, welche mit den Instrumenten des Sets eingebracht werden kann.
Fig. 8 ist eine perspektivische Darstellung eines erfindungsgemäßen Ratschengriffes.
Fig. 9 ist eine Schnittansicht des Ratschengriffes.
Fig. 10 ist eine perspektivische Darstellung der Hülse mit einem Zahnrad, welche Bestandteil des Ratschenmechanismus ist.
Fig. 11 ist eine perspektivische Schnittansicht der Hülse
Fig. 12 zeigt in einer perspektivischen Darstellung die Hülse zusammen mit der Sperrklinke.
Fig. 13 ist eine perspektivische Explosionsdarstellung der Bestandteile des Handgriffs.
Fig. 14 ist eine perspektivische Detaildarstellung, in welche die Ausnehmung, in welcher der Ratschenmechanismus angeordnet ist, zu erkennen ist.
Fig. 15 ist eine Ansicht eines weiteren Griffes, welcher keinen Ratschenmechanismus aufweist.
Fig. 16 ist eine Ansicht eines Ausführungsbeispiels eines Griffes mit einer Zugangsnadel.
Fig. 17 ist eine Schnittansicht des Griffes mit Nadel gemäß Fig. 16.
Fig. 18 zeigt den Griff in einer Detailansicht.

### Detaillierte Beschreibung der Zeichnungen

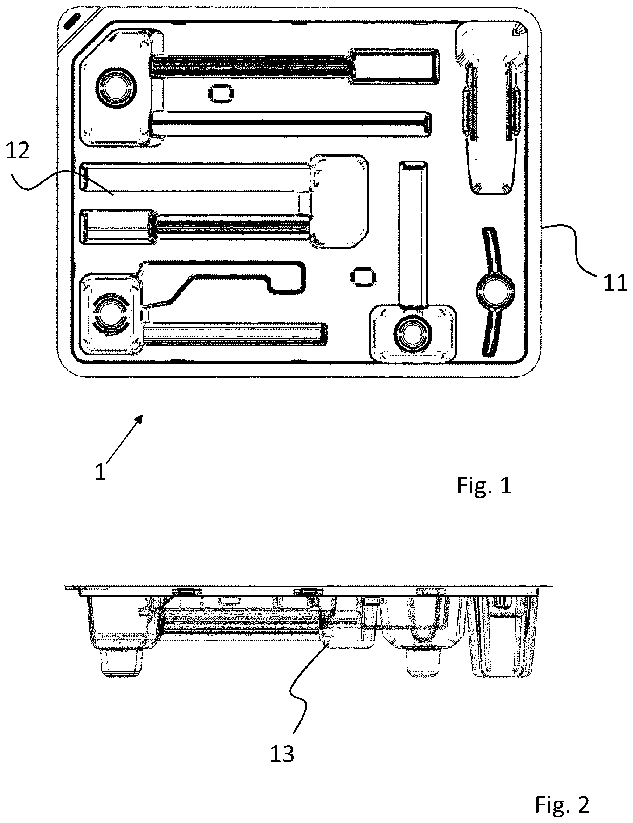

Fig. 1 ist eine Draufsicht auf ein Set 1 zur dorsalen Wirbelfusion, welches zumindest ein Teil der dafür verwendeten Instrumente umfasst.

Das Set ist als Blisterverpackung 11 ausgebildet, welche eine abreißbare, transparente Folie 12 umfasst.

Wie in der Seitenansicht gemäß Fig. 2 zu erkennen, umfasst die Blisterverpackung eine Formschale 13, die für die einzelnen Instrumente Mulden umfasst.

So kann eine übersichtliche Anordnung mit definierter Position der Einzelinstrumente auf einfache Weise bereitgestellt werden.

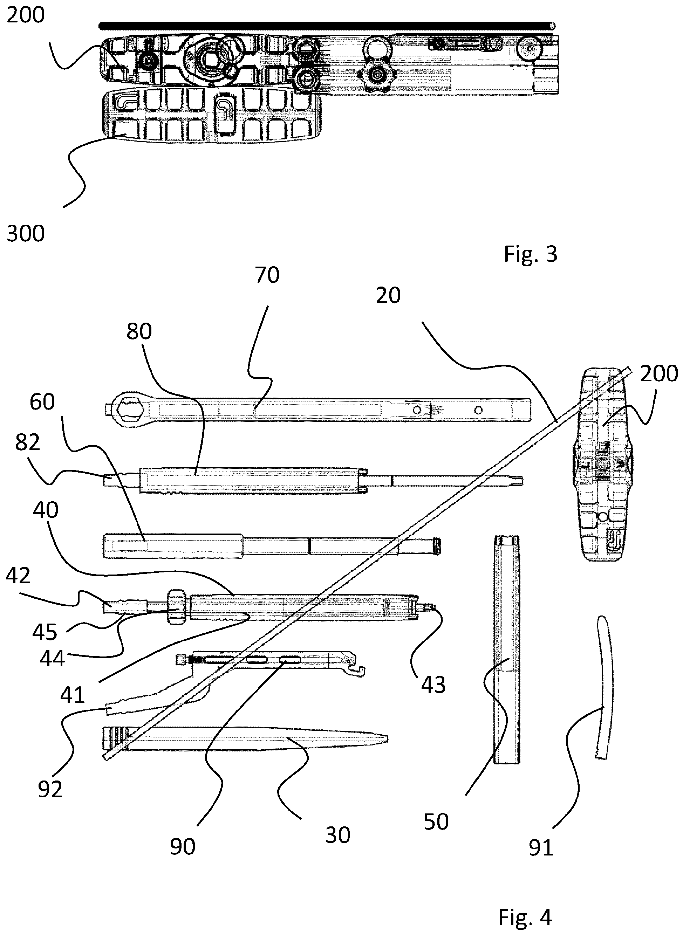

Fig. 3 ist eine Seitenansicht auf die Schmalseite des Sets.

Zu erkennen ist, dass in einer der Mulden der Formschale zwei Griffe, nämlich ein Griff mit einem Ratschenmechanismus 200 und ein weiterer Griff 300 ohne einen Ratschenmechanismus übereinander angeordnet sind.

Je nachdem, welches Instrument gerade angekoppelt ist und je nach Präferenz des Anwenders, kann einer der beiden Griffe 200 oder 300 verwendet werden.

Fig. 4 zeigt in der Draufsicht die in dem Set vorhandenen Instrumente.

Das Set umfasst in diesem Ausführungsbeispiel neben dem Griff 200 einen Führungsdraht 20, welcher über eine Zugangsnadel eingesetzt wird. Die Zugangsnadel ist in diesem Ausführungsbeispiel nicht Teil des Sets, da die meisten Chirurgen eine besondere Präferenz für eine bestimmte Zugangsnadel haben.

Weiter umfasst das Set einen Dilator 30, welcher auf den Führungsdraht 20 aufgeschoben werden kann und mit welchem das Weichgewebe aufgeweitet werden kann.

Zum Einbringen der Fusionsschrauben umfasst das Set den Schraubendreher 40. Der Schraubendreher 40 umfasst eine Hülse 41 mit einem Griff 44, in welchem der Antrieb 42 zum Befestigen an dem Griff 200, der mit dem Schraubentrieb 43 gekoppelt ist, drehbar gelagert ist.

Der Antrieb 42 ist insbesondere polygonförmig, z.B. vierkantförmig ausgebildet, um so formschlüssig durch die entsprechend geformte Aufnahme des Griffes angetrieben zu werden.

Angrenzend zum Antrieb 42 ist eine Nut 45 angeordnet, in welcher ein Rastmittel, bei diesem Set ein federnder Haken des Griffes 200 einrastet und so den Schraubendreher axial sichert.

Zum Verwenden des Schraubendrehers 40 wird dieser mit dem Antrieb 42 in den Griff 200 eingesetzt.

Der Schraubendreher 40 kann werkzeuglos eingesetzt und wieder abgenommen werden.

Weiter umfasst das Set Führungshülsen 50, welche mit den Fusionsschrauben gekoppelt werden können und über die insbesondere die Fusionsschrauben ausgerichtet werden können. Über einen in die Tulpe der Schraube einbringbaren Distraktionsgriff 60 kann die Position der Fusionsschrauben verändert werden.

Der Schraubendreher 80 dient dem Einbringen eines Verschlusses, mit welcher der Fusionsstab mit der Schraube verbunden wird. Auch der Schraubendreher 80 ist mittels eines Antriebs 82 mit dem Griff 200 koppelbar.

Weiter umfasst das Set einen kombinierten Gegenhalter/Flankenbrecher 70, mit welchem zum einen die Führungshülsen 50 in Position gehalten werden können und mit welchen zum anderen am Ende der Operation die Flanken der Tulpe der Fusionsschraube abgebrochen werden können.

Die in dem Set enthaltene Stange 91 kann mit dem Stangenhalter 90 verbunden werden, dient aber nur dem Test, ob die Fusionsschrauben richtig ausgerichtet sind.

Über den Stangenhalter 90 werden sodann die Fusionsstangen eingebracht.

Der Stangenhalter 90 umfasst ebenfalls einen Antrieb 92 und kann so mit dem Griff 200 gekoppelt werden.

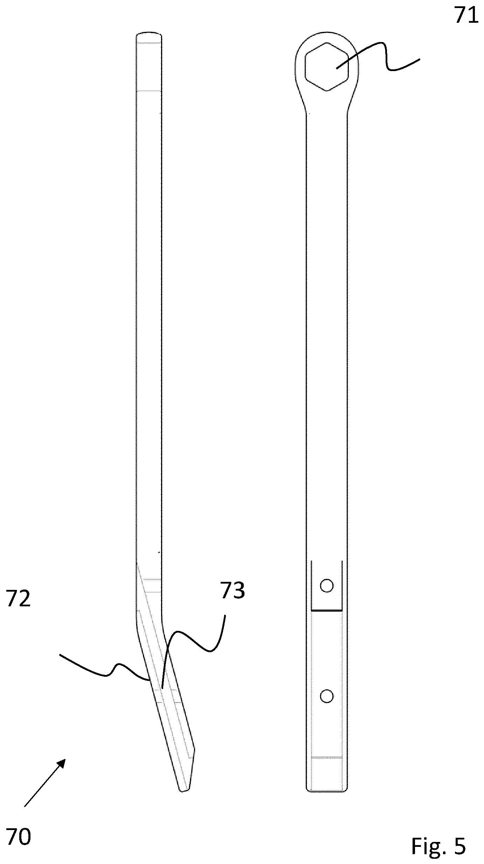

Fig. 5 ist eine Ansicht des kombinierten Gegenhalters/Flankenbrechers 70.

Dieser umfasst an einer Seite einen Schlüssel 71, welcher insbesondere als Sechskant ausgebildet ist. Dieser kann als Gegenhalter für die in Fig. 3 dargestellten Führungshülsen 50 verwendet werden.

Auf der gegenüberliegenden Seite umfasst der kombinierte Gegenhalter/Flankenbrecher 70 ein abgewinkeltes Kopfstück 72, welches einen Kanal 73 umfasst.

Mit dem Kanal 73 kann das Werkzeug auch auf die Flanke der Fusionsschraube geschoben werden und im Anschluss kann die Flanke abgebrochen werden.

Der kombinierte Gegenhalter/Flankenbrecher ist vorzugsweise als Kunststoff, insbesondere als einstückiges Kunststoffspritzgussbauteil ausgebildet.

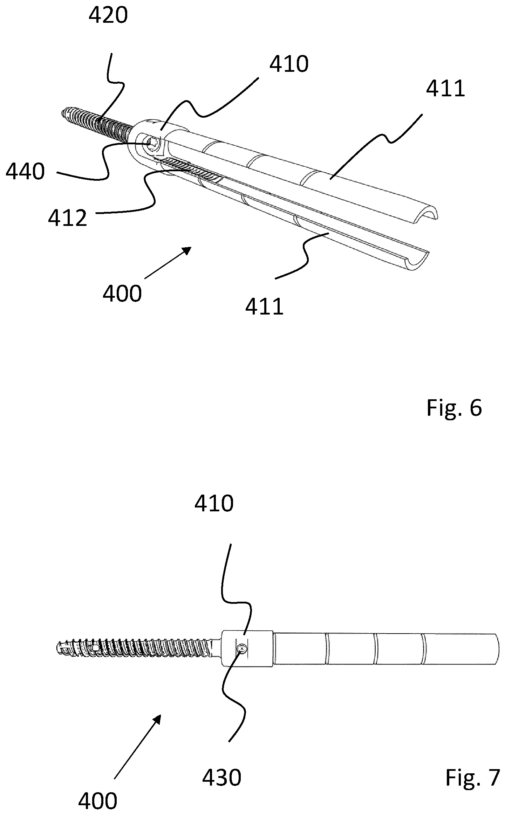

Fig. 6 und Fig. 7 sind perspektivische Ansichten einer Schraube 400, welche mit den Instrumenten des erfindungsgemäßen Sets verarbeitet werden kann.

Die Schraube 400 umfasst eine Tulpe 410 mit Flanken 411.

Die Flanken 411 können segmentiert ausgebildet sein. So können beispielsweise Teilstücke der Flanken 411 abgebrochen werden, um die Höhe der Flanken 411 anzupassen.

Weiter umfasst die Tulpe 410 ein Gewinde 412, in welches die in Fig. 4 dargestellten Distraktionsgriffe (Bezugszeichen 60) eingebracht werden können.

In der Tulpe 410 angeordnet ist der Antrieb 440 der Schraube 400, über die das Gewinde 420 in den Knochen eingedreht werden kann.

Der Antrieb 440 kann beispielsweise als Sechskant oder als Innensechsrund ausgebildet sein.

Zum Einbringen der Schraube 400 in den Knochen wird der in Fig. 4 dargestellte Schraubendreher mit dem Bezugszeichen 40 verwendet.

Wie in der Ansicht gem. Fig. 7 zu erkennen, umfasst die Tulpe 410 Durchgangslöcher 430, durch die die Stangen zur Fusion geschoben werden können.

Über einen hier nicht dargestellten Verschluss, welcher in die Tulpe 410 mittels des in Fig. 4 dargestellten Schraubendrehers mit dem Bezugszeichen 80 eingedreht werden kann, wird die Stange zur Fusion der Wirbelkörper befestigt.

Im Anschluss werden die Flanken 411 mit dem in Fig. 5 dargestellten Flankenbrecher abgebrochen.

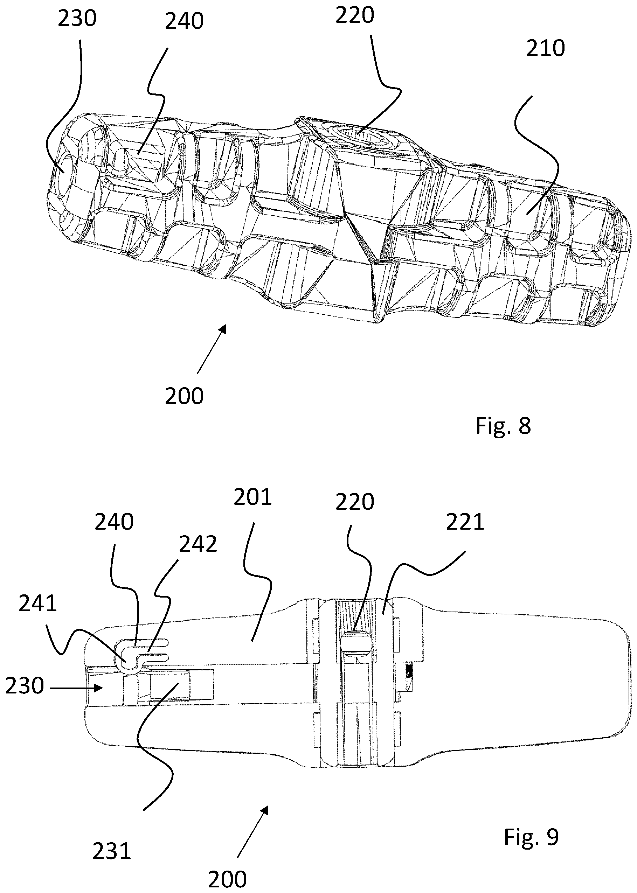

Fig. 8 ist eine perspektivische Ansicht eines Griffes 200, welcher einen Ratschenmechanismus umfasst.

Der Griff 200 ist länglich ausgebildet und umfasst in diesem Ausführungsbeispiel eine Profilierung 210, mit der er sich leichter fassen lässt.

Der Griff 200 umfasst eine mittig angeordnete Aufnahme 220 für ein Werkzeug, insbesondere für einen Schraubendreher. Das Werkzeug kann von beiden Seiten in die Aufnahme 220 eingesetzt werden.

Weiter umfasst der Griff 200 an seiner Schmalseite eine weitere Aufnahme 230, welche dem Einsatz eines Werkzeugs dient.

Zu erkennen ist in dieser Ansicht ein federnder Haken 240, mit welchem das Werkzeug, welches eine entsprechende Nut aufweist, verrastet wird.

Das Werkzeug kann so werkzeuglos eingesetzt und entnommen werden.

Fig. 9 ist eine Schnittansicht des in Fig. 8 dargestellten Griffes.

Zu erkennen ist die Werkzeugaufnahme 230 mit einem polygonförmigen, insbesondere vierkantförmigen, Abschnitt 231 zum Antrieb des Werkzeugs. Angrenzend zum polygonförmigen Abschnitt 231 ragt der federnde Haken 240 in die Werkzeugaufnahme 230 hinein.

Der polygonförmige Abschnitt 431 besteht aus dem Kunststoff des restlichen Griffes. So kann auf einen aufwendig ausgestalteten Einsatz aus Metall verzichtet werden.

Der federnde Haken 240 umfasst ein Eingriffelement 241, welches in diesem Ausführungsbeispiel an seinem Ende abgerundet ausgebildet ist und welches in eine Nut des eingesetzten Werkzeugs eingreift.

Bei Einsetzen und Entnehmen des Werkzeugs biegt sich der federnde Haken 240 zur Seite. Im eingesetzten Zustand greift der federnde Haken 240 eine Nut des Werkzeugs (45 in Fig. 4).

Über einen abgewinkelten federnden Träger 242, welcher einstückig mit dem Hauptgehäuse 201 des Griffes ausgebildet ist, kann das Eingriffelement 241 einfedern, wenn das Werkzeug eingesetzt oder entnommen wird.

Der federnde Haken 240 ist einstückig mit dem Griff 200 und aus Kunststoff ausgebildet.

Je nach Präferenz kann der Anwender entweder den Griff 200 unter Verwendung der Aufnahme 230 axial entsprechend seiner Haupterstreckungsrichtung zum Werkzeug anordnen oder, unter Verwendung der Aufnahme 220 das Werkzeug mit dem Griff in einer T-förmigen Anordnung koppeln.

Der Griff 200 umfasst einen Ratschenmechanismus, der eine Hülse 221 mit der Aufnahme 220 umfasst.

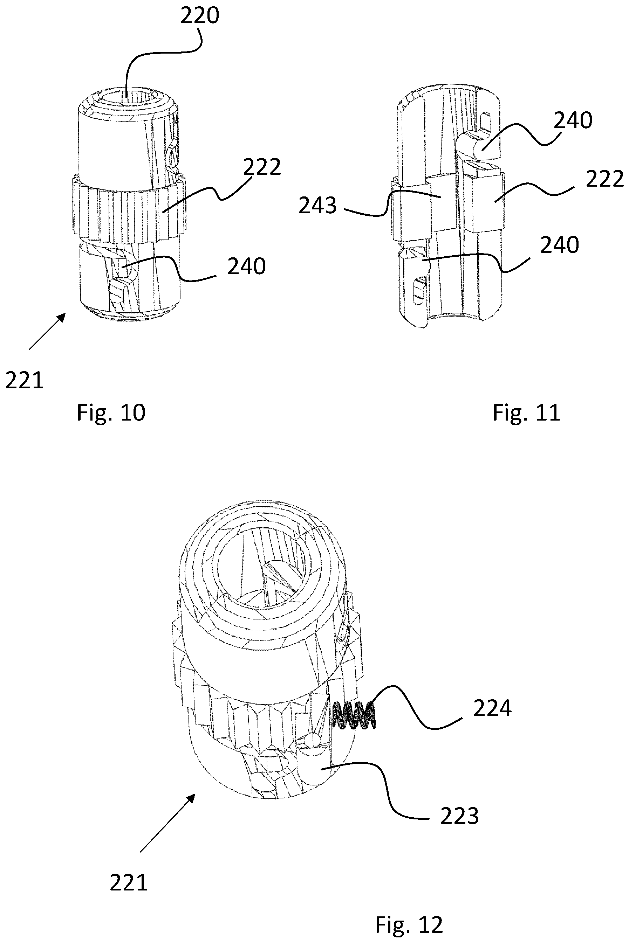

Fig. 10 ist eine perspektivische Ansicht dieser Hülse 221.

Die Hülse 221 ist aus Kunststoff ausgebildet und umfasst als integralen Bestandteil ein Zahnrad 222.

Das Zahnrad 222 ist mittig in der Hülse 221 angeordnet.

Oberhalb und unterhalb des Zahnrades 222 erstrecken sich zwei Bereiche, in denen jeweils, wie in der perspektivischen Schnittansicht gemäß Fig. 11 zu erkennen ist, ein federnder Haken 240 befindet.

Der federnde Haken 240 ist entsprechend des in Fig. 9 dargestellten federnden Hakens ausgebildet.

Der federnde Haken 240 kann insbesondere L-förmig ausgebildet sein.

Die federnden Haken 240, welche integraler Bestandteil der aus Kunststoff bestehenden Hülse 221 sind, ermöglichen, dass ein Werkzeug von beiden Seiten eingesetzt und über die federnden Haken 240 verrastet werden kann.

Mittig umfasst die Hülse 221 einen polygonförmigen Abschnitt 243, welcher dem Antrieb des Werkezeugs dient.

Der polygonförmige Abschnitt 243 liegt innerhalb des Zahnrads 222.

Fig. 12 ist eine perspektivische Ansicht der Hülse 221 sowie einer Sperrklinke 223, welche mittels einer Feder 224 (herein auch Einsatz genannt) gegen das Zahnrad 222 der Hülse gedrückt wird.

Die Sperrklinke 223 ist am Hauptgehäuse angeschlagen.

Die Sperrklinke 223 greift in das Zahnrad 222 und bewirkt so, dass die Hülse 221 über dem Hauptgehäuse nur in eine Richtung verdreht werden kann.

In einer Drehrichtung, in dieser Ansicht entgegen des Uhrzeigersinns, kann die Sperrklinke 223 gegen die Federspannung 224 ausweichen und die Hülse 221 kann gedreht werden.

In der anderen Richtung laufen die Zähen des Zahnrads 222 gegen die Sperrklinke 223, so dass eine Verdrehsicherung gebildet ist.

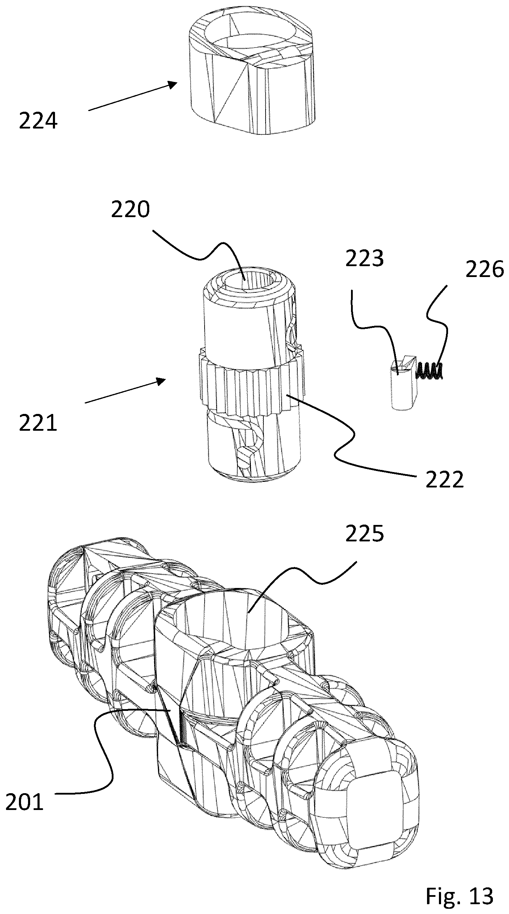

Um die gewünschte Drehrichtung zu ändern, kann das Werkzeug von beiden Seiten in die Hülse 221 eingesetzt werden Fig. 13 zeigt in einer Explosionsdarstellung die Bestandteile des Griffes mit dem Ratschenmechanismus.

Der Griff besteht aus einem Hauptgehäuse 201, welches eine Ausnehmung 225 zur Aufnahme des Ratschenmechanismus aufweist.

In die Ausnehmung 225 wird die Hülse 221 eingesetzt und ist dort drehbar gelagert.

Vor dem Einsetzen der Hülse mit der Aufnahme 220 und dem Zahnrad 222 wird bei Montage des Griffes die Sperrklinke 223 mit der Feder 226 eingesetzt.

Sodann wird die Hülse 221 nebst Sperrklinke 223 und Feder 226 axial durch Einbringen des Einsatzes 224 in die Ausnehmung 225 gesichert.

Der Einsatz 224 kann beispielsweise verrastet oder stoffschlüssig befestigt werden, z. B. mittels Kleben oder Schweißen.

Die Bauteile des Ratschenmechanismuses sind so auf einfache Weise in der Ausnehmung 225 des Hauptgehäuses 201 gesichert.

Vorzugsweise bestehen sämtliche Bauteile des Griffes mit Ausnahme der Sperrklinke 223 und der Feder 226 aus einem Kunststoff. Insbesondere sind diese als Spritzgussbauteile ausgebildet.

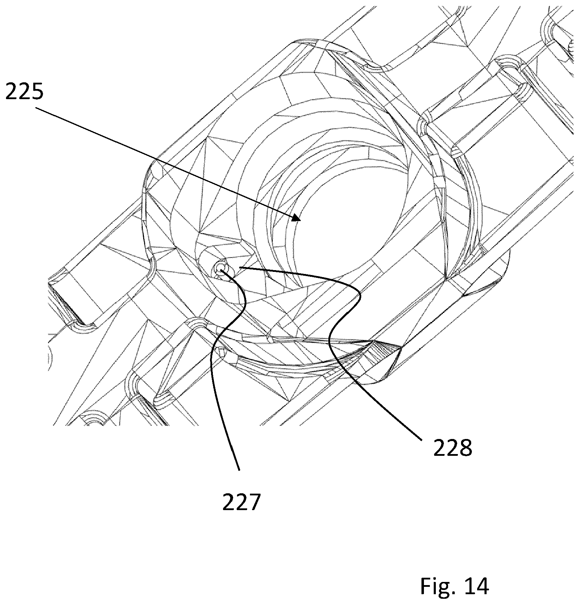

Fig. 14 ist eine perspektivische Detailansicht der Ausnehmung 225.

Zu erkennen ist, dass das Hauptgehäuse innerhalb der Ausnehmung 225 eine Ausnehmung 228 für die Sperrklinke nebst Feder umfasst.

Eine Achse 227 für die Sperrklinke ist als integraler stiftförmiger Bestandteil des Hauptgehäuses ausgebildet.

Die Sperrklinke wird zusammen mit der Feder in die Ausnehmung 228 eingesetzt.

Sodann wird die Hülse (221) in die Ausnehmung 225 gesetzt und die Montage durch Einsetzen des Einsatzes 224 abgeschlossen.

Fig. 14 zeigt in einer Seitenansicht einen Griff 300, welcher keinen Ratschenmechanismus umfasst.

Auch dieser Griff lässt sich sowohl T-förmig als auch axial ausgerichtet mit dem Werkzeug koppeln.

Hierzu umfasst der Griff 300 die Aufnahme 220, in welche das Werkzeug, beispielsweise der Schraubendreher, eingesetzt werden kann.

Ein federnder Haken 240, welcher integraler Bestandteil des Hauptgehäuses 201 des Griffes ist, ermöglicht ein werkzeugloses Verrasten des Griffes.

Weiter umfasst der Griff 300 entsprechend des zuvor dargestellten Griffes mit dem Bezugszeichen 200 an seiner Schmalseite eine Aufnahme 230 für ein Werkzeug.

Auch in dieser Aufnahme wird das eingesetzt Werkzeug mit einem federnden Haken 240, welcher seitlich in eine Nut greift, verrastet.

Zumindest einer der federnden Haken kann, wie hier dargestellt, seitlich offen ausgebildet sein.

Dies ermöglicht zum einen eine gute Entformbarkeit beim Spritzguss.

Zum anderen wird so eine optische Kontrolle ermöglicht, ob der federnde Haken 240 auch in die Nut des Werkzeugs eingreift.

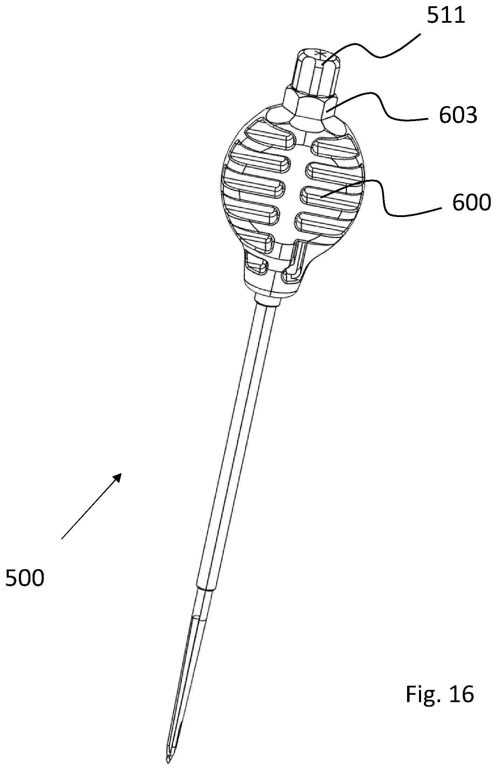

Fig. 16 ist eine Ansicht einer Ausführungsform einer Zugangsnadel 500 mit einem Griff 600.

Der Griff 600 ist derart ausgebildet, von der Seite gegriffen zu werden. Proximal umfasst der Griff 600 eine Kupplung 603 für einen Halter (siehe z.B. Fig. 5). So kann der Griff 600 unter Röntgenkontrolle auch außerhalb des Strahlengangs geführt werden. Die Kupplung 603 kann beispielsweise als Polygon, z.B. Sechskant, ausgebildet sein.

Eine Innnennadel 510 kann über einen proximalen Halter 511 herausgezogen werden.

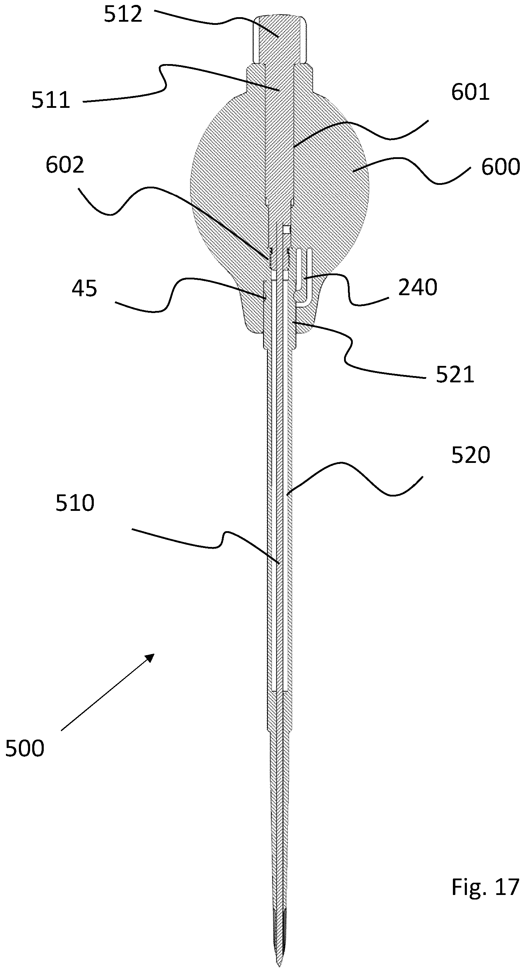

Fig. 17 ist eine Schnittansicht der Zugangsnadel 500. Dies umfasst eine Hohlnadel 520 mit einer Innennadel 510.

Der Griff 600 umfasst eine Durchführung 601 für die Nadeln 510, 520.

Die Hohlnadel 520 ist im distalen Endbereich des Griffes 600 verrastet. Ein Einsatz 521, der vorzugsweise gegenüber dem Griff 600 verdrehgesichert ist, umfasst eine Nut 45, in die ein federnder Haken 240 des Griffes 600 eingreift.

Der federnde Haken 240 ist integraler Bestandteil des Griffes 600 aus Kunststoff und kann insbesondere entsprechend vorstehend beschriebener Ausführungsbeispiele ausgebildet sein. Insbesondere ist der Haken 240 von einer Seite her offen zugänglich.

Proximal nach der Hohlnadel 520 erstreckt sich der Halter 511 der Innennadel 510 durch die Durchführung 601. Der Halter 511 ist über ein Gewinde 602 in der Durchführung 601 mit dem Griff 600 verbunden.

Am proximalen Kopfstück 512 kann der Halter 511 zusammen mit der Innennadel 510 herausgeschraubt und die Innennadel 510 so entnommen werden.

Die Außennadel 520 kann Ihrerseits durch Lösen der Verrastung distal herausgezogen werden.

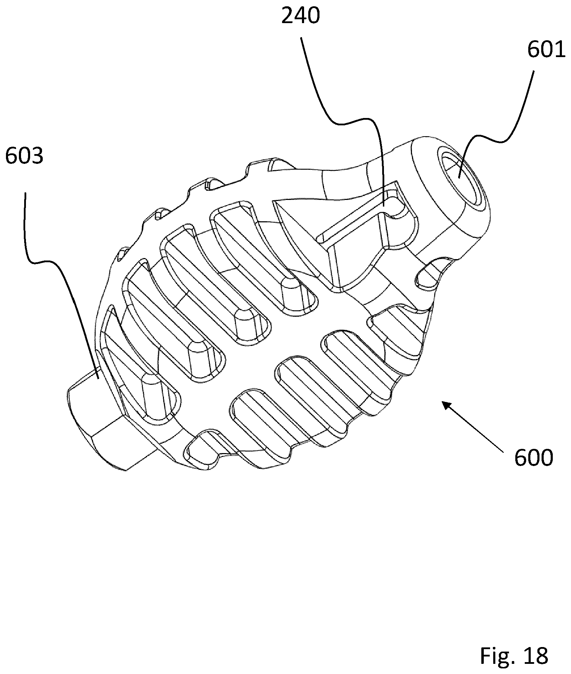

Fig. 18 ist eine perspektivische Ansicht nur des Griffes 600. Der Griff 600 ist als monolithischer Körper aus einem faserverstärkten Kunststoff, einschließlich des Hakens 240 und der Kupplung 603, ausgebildet.

Durch die Erfindung konnte eine Einweginstrumentarium zur dorsalen Wirbelfusion bereitgestellt werden, welches preiswert herzustellen ist und eine gute Handhabbarkeit und Flexibilität ermöglicht.

### Bezugszeichenliste

1 Set zur dorsalen Wirbelfusion
11 Blisterverpackung
12 transparente Folie
13 Formschale
20 Führungsdraht
30 Dilator
40 Schraubendreher
41 Hülse
42 Antrieb
43 Schraubenantrieb
44 Griff
45 Nut
50 Führungshülse
60 Distraktionsgriff
70 kombinierter Gegenhalter/Flankenbrecher
71 Schlüssel
72 Kopfstück des Flankenbrechers
73 Kanal
80 Schraubendreher (für Verschluss)
90 Stangenhalter (für Fusionsstab)
91 Stange
92 Antrieb
200 Griff
201 Hauptgehäuse
210 Profilierung
220 Aufnahme
221 Hülse
222 Zahnrad
223 Sperrklinke
224 Einsatz
225 Ausnehmung
226 Feder
227 Achse
228 Ausnehmung
230 Aufnahme
231 polygonförmiger Abschnitt
240 Haken
241 Eingriffelement
242 federnder Träger
243 polygonförmiger Abschnitt
300 Griff
400 Schraube
410 Tulpe
411 Flanke
412 Gewinde
420 Gewinde
430 Durchgangsloch
440 Antrieb
500 Zugangsnadel
510 Innennadel
511 Halter
512 Kopfstück
520 Hohlnadel
521 Einsatz
600 Griff
601 Durchführung
602 Gewinde
603 Kupplung

## Patentansprüche

1. Set zur dorsalen Wirbelfusion (1), umfassend zumindest einen abnehmbaren Handgriff (200) aus Kunststoff sowie zumindest einen mit dem abnehmbaren Handgriff (200) werkzeuglos koppelbaren Schraubendreher (40), wobei eine Werkzeugaufnahme (220) des Handgriffes (200) für den Schraubendreher (40) aus Kunststoff besteht, wobei der Antrieb (42) des Schraubendrehers (40) mittels eines L-förmig ausgebildeten federnden Hakens (240) aus Kunststoff, welcher integraler Bestandteil des Handgriffes (200) ist und in die Werkzeugaufnahme (230) hineinragt, verrastbar ist, **dadurch gekennzeichnet, dass** der Handgriff (200) als Ratschengriff ausgebildet ist, der einen nicht umschaltbaren Ratschenmechanismus umfasst, wobei zum Ändern der Drehrichtung der Schraubendreher (40) von zwei verschiedenen Seiten in die Werkzeugaufnahme (220) einrastbar ist.

2. Set zur dorsalen Wirbelfusion (1) nach dem vorstehenden Anspruch, **dadurch gekennzeichnet, dass** der Handgriff aus einem Polymer, insbesondere aus einem Thermoplast, insbesondere aus Polyacrylamid, besteht.

3. Set zur dorsalen Wirbelfusion (1) nach einem der vorstehenden Ansprüche, **dadurch gekennzeichnet, dass** der Handgriff (200) als länglich ausgebildeter Griff (200) ausgestaltet ist und sowohl an einer Langseite als auch an einer Schmalseite eine Werkzeugaufnahme (220, 230) umfasst.

4. Set zur dorsalen Wirbelfusion (1) nach einem der vorstehenden Ansprüche, **dadurch gekennzeichnet, dass** der Ratschenmechanismus ein Kunststoffzahnrad (222) umfasst, in welches eine Sperrklinke (223) greift, die eine Drehung des Kunststoffzahnrads (222) in eine Drehrichtung blockiert.

5. Set zur dorsalen Wirbelfusion (1) nach dem vorstehenden Anspruch, **dadurch gekennzeichnet, dass** das Zahnrad (222) integraler Bestandteil der Werkzeugaufnahme (220) ist.

6. Set zur dorsalen Wirbelfusion (1) nach einem der vorstehenden Ansprüche, weiter umfassend zumindest auf eine Knochenschraube aufsetzbare Führungshülse (50), zumindest einen Dilatator (30), einen Gegenhalter für eine Führungshülse (50), einen Halter für einen Fusionsstab und/oder einen Flankenbrecher (70) für die Tulpe (410) einer Fusionsschraube.

7. Set zur dorsalen Wirbelfusion (1) nach dem vorstehenden Anspruch, **dadurch gekennzeichnet, dass** der Flankenbrecher (70) für die Tulpe (410) der Fusionsschraube den Gegenhalter für die Führungshülse (50) umfasst.

8. Set zur dorsalen Wirbelfusion (1) nach einem der vorstehenden Ansprüche, **dadurch gekennzeichnet, dass** das Set als steriles Einwegset mit einer Aufreißverpackung ausgebildet ist.

9. Set zur dorsalen Wirbelfusion (1), weiter umfassend eine Nadel, insbesondere eine Zugangsnadel (500), wobei die Nadel eine Nut (Nut) zum Eingriff des federnden Hakens aufweist, insbesondere des federnden Hakens (240) eines weiteren Griffes für die Nadel.

10. Handgriff (200), ausgebildet für ein Set zur dorsalen Wirbelfusion (1) nach einem der vorstehenden Ansprüche.

11. Handgriff (200) nach dem vorstehenden Anspruch, **dadurch gekennzeichnet, dass** der Handgriff (200) eine Durchführung für eine Nadel, insbesondere eine Zugangsnadel (500), aufweist.

12. Handgriff (200) nach dem vorstehenden Anspruch, **dadurch gekennzeichnet, dass** der Handgriff (200) auf einer proximalen Seite eine Kupplung (603) für eine Verlängerung, insbesondere eine abgewinkelte Verlängerung aufweist.

## Claims

1. A set for dorsal spinal fusion (1), comprising at least one detachable handle (200) made of plastics material and at least one screwdriver (40) that can be coupled to the detachable handle (200) without the use of tools, wherein the handle (200) has a receptacle (220) for the screwdriver (40), which is made of plastics material,
wherein the drive (42) of the screwdriver (40) looks the tool in place by an L-shaped hook (240) of plastics material which is formed integrally with the main body of the handle (200) and which protrudes into the tool receptacle (230),
**characterized in that** the handle (200) is in the form of a ratchet handle, which comprises a rachet mechanism which is designed not to be switchable, wherein, in order to change the direction of rotation, the screwdriver (40) can be locked into the receptacle (220) from two different sides.

2. The set for dorsal spinal fusion (1) of the preceding claim, **characterized in that** the handle is made of a polymer, in particular a thermoplastic material, in particular polyacrylamide.

3. The set for dorsal spinal fusion (1) as claimed in any of the preceding claims, **characterized in that** the handle (200) is in the form of an elongated handle (200) and has a tool receptacle (220, 230) on both its long side and its narrow end.

4. The set for dorsal spinal fusion (1) as claimed in any of the preceding claims, **characterized in that** the handle (200) comprises a ratchet mechanism including a plastic gear (222) in which a pawl (223) engages, which blocks rotation of the plastic gear (222) in one direction of rotation.

5. The set for dorsal spinal fusion (1) of the preceding claim, **characterized in that** the gear is (222) an integral part of the tool receptacle (220).

6. The set for dorsal spinal fusion (1) as claimed in any of the preceding claims, further comprising at least one guide sleeve (50) that can be fitted onto a bone screw, at least one dilator (30), a counter holder for a guide sleeve (50), a holder for a fusion rod, and/or a flank breaker (70) for the tulip (410) of a fusion screw.

7. The set for dorsal spinal fusion (1) of the preceding claim, **characterized in that** the flank breaker (70) for the tulip (410) of the fusion screw comprises the counter holder for the guide sleeve (50).

8. The set for dorsal spinal fusion (1) as claimed in any of the preceding claims, **characterized in that** the set is in the form of a sterile single-use set comprising a tear-open packaging.

9. The set for dorsal spinal fusion (1), further comprising a needle, in particular an access needle (500), wherein the needle has a groove for engagement of the resilient hook, in particular of the resilient hook (240) of a further handle for the needle.

10. A handle (200) being embodied for a set for dorsal spinal fusion (1) as claimed in any of the preceding claims.

11. The handle (200) of the preceding claim, **characterized in that** the handle (200) has a passage for a needle, in particular for an access needle (500).

12. The handle (200) of the preceding claim, **characterized in that** the handle (200) has a coupling (603) for an extension, in particular for an angled extension, on a proximal end thereof.

## Revendications

1. Ensemble pour fusion vertébrale dorsale (1), comprenant au moins une poignée amovible (200) en matière plastique ainsi qu'au moins un tournevis (40) qui peut être couplé à la poignée amovible (200) sans outil, un porte-outil (220) de la poignée (200) pour le tournevis (40) étant en matière plastique, l'entraînement (42) du tournevis (40) pouvant être enclenché au moyen d'un crochet élastique (240) en matière plastique, réalisé en forme de L, qui fait partie intégrante de la poignée (200) et fait saillie dans le porte-outil (230), **caractérisé en ce que** la poignée (200) est conçue comme une poignée à cliquet qui comprend un mécanisme à cliquet non réversible, le tournevis (40) pouvant être encliqueté dans le porte-outil (220) de deux côtés différents afin de changer le sens de rotation.

2. Ensemble pour fusion vertébrale dorsale (1) selon la revendication précédente, **caractérisé en ce que** la poignée est constituée d'un polymère, en particulier d'un thermoplastique, notamment de polyacrylamide.

3. Ensemble pour fusion vertébrale dorsale (1) selon l'une quelconque des revendications précédentes, **caractérisé en ce que** la poignée (200) est conçue comme une poignée (200) de forme allongée et comprend un porte-outil (220, 230) aussi bien sur un côté long que sur un côté étroit.

4. Ensemble pour fusion vertébrale dorsale (1) selon l'une quelconque des revendications précédentes, **caractérisé en ce que** le mécanisme à cliquet comprend une roue dentée en plastique (222) dans laquelle s'engage un cliquet (223) qui bloque la rotation de la roue dentée en plastique (222) dans un sens de rotation.

5. Ensemble pour fusion vertébrale dorsale (1) selon la revendication précédente, **caractérisé en ce que** la roue dentée (222) fait partie intégrante du porte-outil (220).

6. Ensemble pour fusion vertébrale dorsale (1) selon l'une quelconque des revendications précédentes, comprenant en outre au moins une douille de guidage (50) pouvant être placée sur une vis osseuse, au moins un dilatateur (30), un contre-support pour une douille de guidage (50), un support pour une tige de fusion et/ou un outil de rupture des flancs (70) pour la tulipe (410) d'une vis de fusion.

7. Ensemble pour fusion vertébrale dorsale (1) selon la revendication précédente, **caractérisé en ce que** l' outil de rupture des flancs (70) pour la tulipe (410) de la vis de fusion comprend le contre-support pour la douille de guidage (50).

8. Ensemble pour fusion vertébrale dorsale (1) selon l'une quelconque des revendications précédentes, **caractérisé en ce que** l'ensemble est conçu comme un ensemble stérile à usage unique avec un emballage déchirable.

9. Ensemble pour fusion vertébrale dorsale (1), comprenant en outre une aiguille, en particulier une aiguille d'accès (500), l'aiguille présentant une rainure (45) pour l'engagement du crochet élastique, en particulier du crochet élastique (240) d'une autre poignée pour l'aiguille.

10. Poignée (200) conçue pour un ensemble pour fusion vertébrale dorsale (1) selon l'une quelconque des revendications précédentes.

11. Poignée (200) selon la revendication précédente, **caractérisée en ce que** la poignée (200) présente un passage pour une aiguille, en particulier une aiguille d'accès (500).

12. Poignée (200) selon la revendication précédente, **caractérisée en ce que** la poignée (200) présente, sur un côté proximal, un accouplement (603) pour une extension, en particulier une extension coudée.
